# EUROPEAN PATENT SPECIFICATION

(11) **EP 4 436 655 B1**
(45) Date of publication and mention of the grant of the patent: **14.10.2026**
(21) Application number: 22821988.7
(22) Date of filing: 23.11.2022
(51) Int. Cl.: A61N 1/365, A61N 1/375

(54) **PACEMAKER AND OPERATION METHOD OF SUCH PACEMAKER**
SCHRITTMACHER UND BETRIEBSVERFAHREN FÜR EINEN SOLCHEN SCHRITTMACHER
STIMULATEUR CARDIAQUE ET PROCÉDÉ DE FONCTIONNEMENT D'UN TEL STIMULATEUR CARDIAQUE

(30) Priority: 23.11.2021 US 202163282220 P; 06.12.2021 EP 21212413
(43) Date of publication of application: 02.10.2024
(73) Proprietor: BIOTRONIK SE & Co. KG, 12359 Berlin (DE)
(72) Inventor: QU, Min, Portland, Oregon 97223 (US); KIBLER, Andrew B., Lake Oswego, Oregon 97035 (US)
(74) Representative: Biotronik Corporate Services SE
(86) International application number: PCT/EP2022/082967
(87) International publication number: WO 2023/094442

(56) References cited:
- WO-A1-2020/088825
- US-A- 5 423 867
- US-A- 5 514 162
- US-B1- 8 065 001

## Description

The invention is generally directed to a pacemaker for a patient's heart and an operation method of such pacemaker, a respective computer program product and computer readable data carrier.

A pacemaker (or artificial pacemaker) for a patient's heart is a medical device that generates electrical pulses delivered by electrodes connected to or fixed at the pacemaker to cause the heart muscle chambers (i.e. the atria and/or the ventricles) of the patient to contract and therefore pump blood. By doing so this device replaces and/or regulates the function of the electrical conduction system of the heart. One purpose of a pacemaker is to maintain an adequate heart rate, either because the heart's natural pacemaker is not fast enough, or because there is a block in the heart's electrical conduction system. Additionally or alternatively, the pacemaker may stimulate different positions within the ventricles to improve their synchronization of the ventricles or provide defibrillation functions in order to treat life-threatening arrhythmias. Modern pacemakers are externally programmable and allow a health care provider (HCP) to select the optimal pacing mode(s) for individual patients.

A conventional pacemaker comprises a controlling and generator device comprising a processing unit and a power source external of the patient's heart and electrodes that are implanted within the heart's muscle. The electrodes are connected via leads and a header located at the device to the device. In most cases the device is implanted transcutaneous in the front of the chest in the region of the left or right shoulder. An implantable intra-cardiac pacemaker (also known as implantable leadless pacemaker - ILP), is well known miniaturized pacemaker which is entirely implanted into a heart's ventricle (V) or atrium (A) of a patient. ILPs are considered the future of cardiac pacing. Alternative or additional functions of conventional or intra-cardiac pacemakers comprise providing other electrical or electromagnetic signals to the heart or its surrounding tissue and sensing electrical or electromagnetic signals (e.g. signals from electrical depolarization fields) or other physiological parameters of the heart and/or its surrounding tissue such as the intrinsic (i.e. the heart's natural) atrial contraction or the intrinsic (i.e. the heart's natural) ventricular contraction. Due to the highly restricted size, an ILP has a small battery capacity. As a selfcontained implantable device, the size of the ILP is as small as 1 cm³, and the battery volume and capacity in the leadless pacemaker is significantly lower (less than 1/10) than for the conventional pacemaker. In order to ensure the longevity of the ILP is comparable to the conventional pacemaker (around or above 10 years), it is critical to keep the current consumption of all the modules/units of the ILP minimal.

In a VDD mode, which may be used in a conventional pacemaker or in an ILP, the pacemaker synchronizes ventricular pacing with the intrinsic ventricular or atrial timing by sensing when ventricular or atrial contractions occur. The ventricular pacing - if necessary - is then calculated based on a pacing rate, wherein in an activity-based rate responsive pacing algorithm the actual pacing rate considers the current activity of the patient. Such algorithm realizes the finding that the pacing rate needs to be increased to meet the associated higher metabolic requirements if the pacemaker-dependent patient is active. Accordingly, if the patient is not active, the pacing rate needs to be decreased.

In the activity-based rate responsive algorithm of the conventional pacemaker having a motion sensor, a scaling factor, i.e., gain value, is used to convert the physical exertion signal derived from a motion signal of a motion sensor (sensing the patient's activity) into the target exertion-modulated pacing rate. The gain value is patient specific and slowly adjusted automatically over many days to ensure that the generated target rate meets the metabolic requirement of each individual patient. In the conventional pacemaker, when the auto-gain feature is enabled, this rate adaptation algorithm is running continuously, even if the device is inhibited from pacing, to ensure the scaling factor in the rate adaptation algorithm is adjusted to suit the patient's unique needs. Even if the pacemaker is not working in the physical exertion-based rate adaptation mode, the motion sensor and part of the algorithm still run to support the update of the gain value.

The above method causes unnecessary current consumption. In particular, an ILP which has very strict size and power consumption requirements challenges the continuous running of rate adaptation algorithm. The current consumption of the motion sensor and the rate adaptation algorithm has a significant impact on the lifetime. Further, the known method is susceptible to random noise in the activity level. If there is an unexpected or abnormal day with extremely low or high activity, i.e., random noise, the auto-gain value will be adjusted accordingly. And in the following day(s), the gain value corresponding to the abnormal noise will be applied in the rate adaptation algorithm, until the gain value is re-adjusted.

US 5 514 162 A discloses a rate-responsive pacemaker that measures a patient's level of activity and stores the results in an activity level histogram. Tue patient's average level of activity is maintained as a running average. Based on a prescribed base pacing rate, a prescribed maximum pacing rate, the average level of activity, and the measured levels of activity stored in the activity level histogram, the rate-responsive pacemaker automatically calculates the slope of the pacemaker transfer function. An activity deviation histogram is also maintained. Analysis of the activity deviation histogram allows the pacemaker to determine if the patient was usually inactive for an extended period of time, and to inhibit an automatic slope calculation under such conditions. If a patient desires to reach a prescribed target heart rate during exercise, the slope may be calculated using the target heart rate and a prescribed fraction of time each week that the patient devotes to exercise.

Accordingly, there is the need for a pacemaker which provides activity-based pacing but works with low power consumption.

The above problem is solved with a pacemaker comprising the features of claim 1 and an operation method comprising the features of claim 8 as well as with a computer program product comprising the features of claim 14 and a computer readable data carrier comprising the features of claim 15.

In particular, a pacemaker for a patient's heart is disclosed comprising a processing unit, a detector and a pacing signal generator, wherein the processing unit, the detector and the pacing signal generator are electrically interconnected. The detector is configured to determine activity signals of the patient and to transmit or provide the activity signals to the processing unit. The processing unit is configured to determine a pacing rate based on the currently received activity signals of the detector and on a gain value in an adaption mode or in a stabilized mode, wherein the processing unit is configured to produce a pace control signal based on the determined pacing rate and to transmit or provide it to the pacing signal generator. In the adaption mode the processing unit is configured to continuously or stepwise adapt the gain value to the specific patient, wherein the processing unit is configured to stay in the adaption mode as long as at least one stability criterion is not met and to transition in the stabilized mode if the processing unit identifies that the at least one stability criterion is met. In the stabilized mode the processing unit is configured to use a locked gain value determined based on the most recently adapted gain values for determining the pacing rate.

The processing unit processes signal data received from the detector, for example activity signals which are detected over time. From these signals, the processing unit may determine a motion signal which is explained in detail below.

The pacemaker may be a conventional cardiac pacemaker or an ILP having the general structure as indicated above.

The ILP or the conventional pacemaker may be operated in VDD pacing mode (i.e. a pacing mode in which the ventricle is stimulated according to atrial activity and AV conduction monitoring). In the VDD mode, the pacemaker synchronizes ventricular pacing with the intrinsic atrial timing by sensing when atrial contractions occur. In an ILP that is implanted in the right ventricle, the atrial contraction information can be detected as a far field signal, but with less reliability and accuracy than in a dual chamber pacemaker where there is a lead in the right atrium as well as the right ventricle. In the VDD mode, since there are no atrial paces, the pacemaker is completely reliant on synchronizing to the cardiac conditions rather than being able to control the timing in both chambers as can be done in the DDD pacing mode (in which the atrium an ventricle are paced). The processing unit may be configured to detect an atrial sense event (i.e. a sensed natural atrial contraction) and/or a ventricular sense event (i.e. a sensed natural ventricular contraction), for example by the detector which may be configured to sense electrical and/or electromagnetic signals, e.g. signals from electrical depolarization fields.

With regard to the invention the processing unit is generally regarded as a functional unit of the pacemaker, that interprets and executes instructions comprising an instruction control unit and an arithmetic and logic unit. The processing unit may comprise a microprocessor, a controller, a digital signal processor (DSP), an application specific integrated circuit (ASIC), a field-programmable gate array (FPGA), discrete logic circuitry or any combination thereof. Further, the processing unit may comprise a counter and a clock. The counter may be used to count clock signals of the clock. The counter may be started at each sensed atrial or ventricular event and count the number of clock signals until a ventricular sense event is determined or a ventricular pace signal is provided by the pacing signal generator. The actual pacing rate determined by the processing unit is used to provide a pacing control signal (e.g. the ventricular pace control signal) to the pacing signal generator. It is calculated using the last sensed atrial or ventricular event or the last atrial or ventricular pacing control signal, wherein it may use the clock signals counted by the counter.

Based on the pacing control signal the pacing signal generator produces the electrical pacing signal(s) in order to transfer it to the electrodes which apply the signal(s) to the heart's tissue adjacent to the electrode.

The pacemaker may comprise a data memory which may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device.

The detector comprises an accelerometer, a vibration sensor, an acoustic sensor (including ultrasound) and/or any other mechanical, electric and/or magnetic sensor that is capable to detect the activity of the patient dependent on time (i.e. a motion sensor), e.g. whether the patient moves or moves not, for example lies, sleeps, sits, moves fast or slowly, including exercising. The detector collects the activity signals of the patient and transforms them into electrical signals. Further, the detector may digitize analog signals or smooth them. Some pre-processing steps may be provided by the detector, as well. The time dependent activity signals produced by the detector may be transmitted to the processing unit directly or after a pre-defined time delay. Further, the detector may comprise means to detect ECG signals as indicated above.

The pacing signal generator produces the pacing signals which are then applied via the electrodes to the heart's tissue. The pacing signals are pulses that begin at a desired time point and have a desired intensity and length. Further, the pulse form may be varied. Information on the pacing signals that are necessary to produce the correct pacing signals are provided by the pace control signal of processing unit.

The pacemaker may comprise further modules such as a communication unit for communication with a remote computer and a power supply such as a battery. The communication unit may exchange messages with the external (at least partially extracorporeally) remote computer, for example in one single direction or bidirectionally. The communication may be provided wirelessly via the patient's body and/or the air using electromagnetic waves, for example Bluetooth, WLAN, ZigBee, NFC, Wibree or WiMAX in the radio frequency region, or IrDA or free-space optical communication (FSO) in the infrared or optical frequency region or by wire (electrical and/or optical communication). The remote computer is a functional unit that can perform substantial computations, including numerous arithmetic operations and logic operations without human intervention, such as, for example, a personal mobile device (PMD), a desktop computer, a server computer, clusters/warehouse scale computer or embedded system. The pacemaker's units and components may be contained within a hermetically sealed housing.

In one embodiment the pacemaker comprises electrodes for application of an electrical pacing signal provided by the pacing signal generator. The electrodes are electrically connected to the pacing signal generator via a header of the pacemaker. In one embodiment (i.e. in the case in which the pacemaker is a conventional pacemaker) the electrode may comprise a lead which may be detachably connected to the respective connector at the header. With regard to an ILP one electrode may be located at a distal end of the ILP, close to a fixation member by which the ILP is fixed in the tissue of the patient's heart, for example within the inner tissue of a ventricle. A second electrode may be located at the proximal end of the ILP or a part of the ILP housing that may, for example, serve as counter electrode. Further, the electrodes may be adapted to detect intrinsic ventricular signals or intrinsic atrial signals by picking up electrical potentials. The electrodes may thereby be part of the detector of the pacemaker.

According to the invention the processing unit of a pacemaker that operates for example in a VDD mode, is configured to determine a pacing rate based on the currently received time dependent activity signals of the detector, e.g. raw accelerometer signals provided by the detector as indicated above, and on a gain value. The gain value is a scaling factor that is used as a slope to convert a motion signal derived from the activity signals of the detector into the pacing rate which is thereby adapted to be suitable for the currently sensed activity of the patient. The amplitude of the activity signals over time are first compared with an activity threshold by the processing unit. Only the activities above motion threshold are used to calculate the pacing rate. If the activities are below motion threshold, the pacing rate will not be updated, and the gain value will not be affected. In the next step an integral of the activity signals above the threshold over a pre-determined time period (in the following integration time period), e.g. over 1 second or 1 minute, is calculated, wherein the integral is the motion signal mentioned above. If the activities are below the motion threshold, they signal will not be integrated, and the pacing rate will not be updated. Then, a scaled activity value is derived from the motion signal wherein the motion signal is multiplied by a first component of the gain value (slope) and this product may be added to a second component of the gain value (offset), if applicable. The so calculated scaled activity value is added to a pre-determined basic pacing rate to obtain the activity-based pacing rate for the patient. As indicated above the gain value may comprise two components, namely a slope value and an offset value. Alternatively, the gain value may comprise the slope value only and/or one value for each activity direction of the 3-dimensional space thereby calculating the scaled activity for each direction in space separately. As a further alternative, the gain value may be determined for different time period types, for example a resting day, an exercise day, a sleeping time period, or an active time period. The final value of the scaled activity is the absolute value over the 3 dimensions. In case of different types of time period, the scaled activity is calculated for each type of time period separately. The determined scaled activity is then used to determine the activity-based pacing rate as indicated above. Accordingly, the gain value may be a 1-dimensional or a multi-dimensional value. Further, the gain value may have one component (the slope only) or two components (slope and offset) for each gain value dimension.

During the adaption mode the gain value is continuously or stepwise adapted to the specific patient by the processing unit using the above calculation and based on the below explanation.

In one embodiment the stability criterion is met if an absolute value of a difference of a first average value of the adapted gain values determined within a first time period (in the following also referred to as first window) and a second average value of the adapted gain values determined within a second or the next time period (in the following also referred to as second window) is less than a pre-determined stability threshold value. This means that the stability criterion assesses the delta between the first average value of gain values and the second average values of gain values between two adjacent windows. Therein, in one embodiment the first time period and the second time period are directly adjacent, most recently assessed time periods. The first time period and the second time period may be several hours, one day or several days and may cover a plurality of integration time periods mentioned above. The length of the first time period and the second time period may be identical. The average value may be an arithmetic, geometric or harmonic mean or median value of the gain values determined within the first window or the second window, respectively. Alternatively or additionally, the stability criterion may be met if the pacing rate is updated in the adaptation mode for a pre-defined adaption time period, for example, 60 days. The pre-programmed or user-adaptable (e.g. by a programmer) adaption time period is long enough in order to allow full adaption of the gain value and may cover a plurality of integration time periods. In one embodiment, both stability criteria may continuously be checked and the transition into the stabilized mode is provided by the processing unit if one of both criteria is fulfilled.

In detail, in the adaption mode which is the initial mode after a conventional pacemaker is implanted, the gain value is set to a pre-defined initial value. For safety considerations, in order to avoid pacing at an excessively high rate, the initial value of gain is close to the lowest setting. When the rate adaptation algorithm is running in the adaption mode, the pacing rate is calculated using the activity signal and the actual gain value as indicated above. Accordingly, in the adaption mode the target pacing rate and the gain value are adapted simultaneously. The statistics of the pacing rate are calculated and used to adjust the gain value automatically for every pre-defined time period, for example, every day. Therein, in one embodiment, the duration counter is used to record the duration when the activity rate is above the pre-defined maximum rate threshold. This means the patient's pacing rate is high and can meet the increased metabolic requirement for the period of duration counter. In the balanced scenario, the duration counter should be within a pre-defined range, for example, 30 minutes to 60 minutes each day. If the duration counter is below the pre-defined balance (pacing rate above the maximum rate threshold for too short), the gain is too low and need to be incremented one or more setting steps. On the other hand, if the duration counter is above the pre-defined balance (pacing rate above the maximum rate threshold for too long), the gain is too high and need to be decremented by one or more setting steps. The goal of the adjustment is to make sure the distribution of the pacing rate over a certain period meets a defined balance, a.k.a., a pre-defined rate balance of the duration counter.

If the above explained stability criterion is reached, i.e. the processing unit has indicated that the average gain value is stabilized, for example as described above, the processing unit enters the stabilized mode in which the locked gain value is used to determine the pacing rate from the activity signals provided by the detector. In the stabilized mode the locked gain value may be the most recently determined average gain value (calculated in the adaption mode). In the stability mode the gain value is not changed.

In one embodiment, in the stabilized mode the processing unit is configured to interrupt or reduce detecting the activity signals by the detector, wherein the processing unit is configured to determine the motion signal from the reduced activity signals. For example, the activity signals are determined less frequently by the detector, for example each second minute only. Further, if the activity-based algorithm is currently not used in the pacemaker, the detection of the activity signals by the detector is interrupted. This may be set by a programmer.

In one embodiment, the processing unit transitions from the stabilized mode into the adaption mode if the processing unit receives a respective request, for example by a programmer, and/or if a pre-defined third time period, for example 6 months, is expired since the transition into the stabilized mode. The operation of the adaption mode (after return in this mode) is similar to the first use of the adaption mode after implantation of the pacemaker. However, in this case, as an initial gain value for the adaption mode the locked gain value is used.

The above problem is further solved by an operation method of a cardiac pacemaker, wherein the pacemaker comprises a processing unit, a detector and a pacing signal generator, wherein the processing unit, the detector and the pacing signal generator are electrically interconnected, wherein activity signals of the patient are determined by the detector and transmitted to the processing unit, wherein a pacing rate is determined by the processing unit based on the current activity signals of the detector and based on a gain value in an adaption mode or in a stabilized mode, wherein a pace control signal is produced by the processing unit based on the determined pacing rate and transmitted to the pacing signal generator, wherein in the adaption mode the gain value is continuously or stepwise adapted to the specific patient, wherein the processing unit stays in the adaption mode as long as at least one stability criterion is not met and transitions in the stabilized mode if the processing unit identifies that the at least one stability criterion is met, wherein in the stabilized mode the processing unit uses a locked gain value determined based on the most recently adapted gain values for determining the pacing rate.

As indicated above, the gain value may be a 1-dimensional or multidimensional value, and may have one component or two components.

In one embodiment of the operation method, the stability criterion is met if an absolute value of a difference of a first average value of the adapted gain values determined within a first time period and a second average value of the adapted gain values determined within a second time period is less than a pre-determined stability threshold value, wherein the first time period and the second time period may be directly adjacent, most recently assessed time periods.

In one embodiment, in the stabilized mode detection of the activity signals by the detector are interrupted or reduced by the processing unit, wherein the processing unit determines a motion signal as indicated above from the reduced activity signal.

In one embodiment, as described above, a transition from the stabilized mode into the adaption mode is provided by the processing unit if the processing unit receives a respective request and/or if a pre-defined third time period, e.g. 6 months, is expired since the transition into the stabilized mode.

The above embodiments of the operation method have the same advantages as the above pacemaker. Embodiments of the pacemaker indicated above may be realized in the operation method analogously. It is referred to the above explanation of the pacemaker in this regard.

The above method is, for example, realized as a computer program which comprises instructions which, when executed, cause the processing unit (processor) to perform the steps of the above method (to be executed by the pacemaker, in particular at its processor) which is a combination of above and below specified computer instructions and data definitions that enable computer hardware to perform computational or control functions or which is a syntactic unit that conforms to the rules of a particular programming language and that is composed of declarations and statements or instructions needed for a above and below specified function, task, or problem solution.

Furthermore, a computer program product is disclosed comprising instructions which, when executed by the processing unit, cause the processing unit to perform the steps of the above defined method. Accordingly, a computer readable data carrier storing such computer program product is disclosed.

In this disclosure, an automatic locking method is proposed for the gain value to meet the tight current consumption requirement and still support the automatic adaption of the gain value. Typically, in the adaption mode the gain value will be stabilized after the adaptation algorithm runs for a long enough period. Unless there is a significant change of the activity level after this period, the gain value remains stable with small variations. A method to lock the adapted gain value into the stable value is proposed. By doing this, it is not necessary to run the detector and the adaptation algorithm in the background continuously, thus the longevity of the pacemaker can be extended.

The present invention will now be described in further detail with reference to the accompanying schematic drawing, wherein
- Fig. 1: shows a first embodiment of the pacemaker within a cross section of a patient's heart,
- Fig. 2: depicts a functional block diagram of the pacemaker shown in Fig. 1,
- Fig. 3: shows a flowchart of an embodiment of the inventive operation method, and
- Fig. 4 - 6: depicts diagrams showing the variability of the gain value (in the adaption mode) before and after the stability criterion was reached.

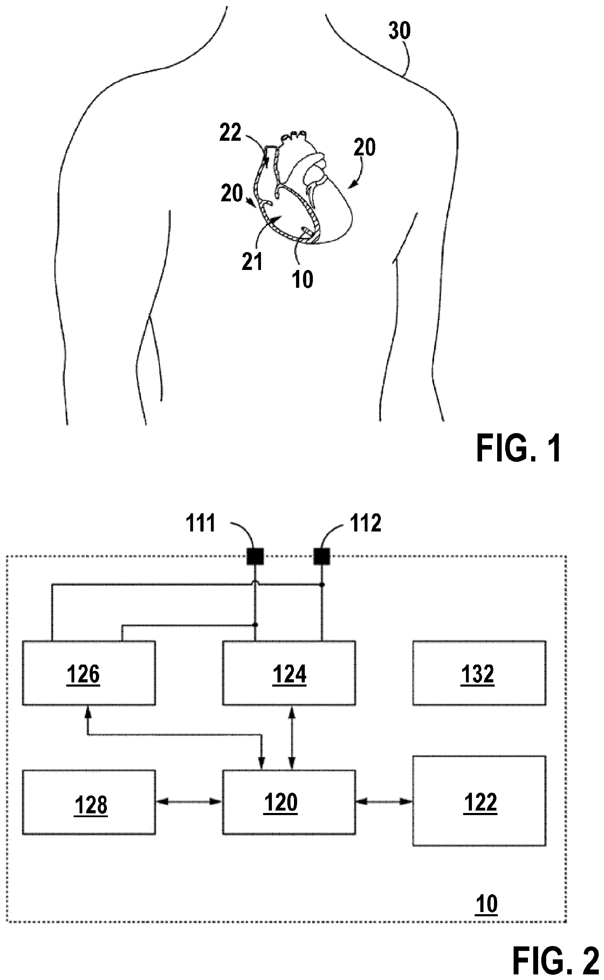

In the following the embodiment of the invention refers to an ILP type pacemaker. However, the invention may analogously be realized in a conventional pacemaker, as well. Fig. 1 shows an example leadless ventricular pacemaker (ILP) 10 implanted within the heart 20 of a patient 30. ILP 10 may be configured to be implanted within the right ventricle 21 of the heart 20 and pace this ventricle in the VDD mode, sense intrinsic ventricular depolarizations and depolarizations of the atria (e.g. the right atrium 22), and inhibit ventricular pacing in response to detected ventricular depolarization. A programmer (not shown) may be used to program ILP 10 and retrieve data from ILP 10.

Fig. 2 shows a functional block diagram of the ILP 10 configured for implantation within ventricle 21 (Fig. 1). In case of a conventional pacemaker the units are contained in a controlling and generator device to which electrodes are connected via leads. The ILP 10 comprises a processing unit 120 with a clock and at least one counter for the clock signals, a data memory 122, a pacing signal generator 124, a detector 126, a communication unit 128, and a power source 132. The power source 132 may include a battery, e.g., a rechargeable or non-rechargeable battery. The power source provides electrical energy to all units and components of the ILP 10, in particular to all units mentioned above and is therefore electrically connected to these units and components. Units included in ILP 10 represent their respective functionality. Similar or identical units and functionality may also be included in the ILP 10. Units of the present disclosure may include any discrete and/or integrated electronic circuit components that implement analog and/or digital circuits capable of producing the functions attributed to the units herein. For example, the units may include analog circuits, e.g., amplification circuits, filtering circuits, and/or other signal conditioning circuits. The units may also include digital circuits, e.g., combinational or sequential logic circuits, memory devices, etc. The data memory 122 may include any volatile, non-volatile, magnetic, or electrical media mentioned above. Furthermore, the processing unit 120 may include instructions that, when executed by one or more processing circuits, cause the units to perform various functions attributed to these units herein. The functions attributed to the units herein may be embodied as one or more processors, hardware, firmware, software, or any combination thereof. Depiction of different features as units is intended to highlight different functional aspects, and does not necessarily imply that such units must be realized by separate hardware or software components. Rather, functionality associated with one or more units may be performed by separate hardware or software components, or integrated within common or separate hardware or software components. Data memory 122 may include computer-readable instructions that, when executed by processing unit 120, cause processing unit 120 to perform the various functions attributed to processing unit 120 herein. Further, data memory 122 may store parameters for these functions, e.g. pacing signal parameters. For example, data memory 122 may store a pre-defined programmable AV delay. The pacing instructions and pacing signal parameters may be updated by the programmer using the communication unit 128. The communication unit 128 may comprise an antenna or a transceiver.

The processing unit 120 may communicate with pacing signal generator 124 and detector 126 thereby transmitting signals. Pacing signal generator 124 and detector 126 are electrically coupled to electrodes 111, 112 of the ILP 10. Detector 126 is configured to monitor signals from electrodes 111, 112 in order to monitor electrical activity of heart 20. Further, the detector 126 comprises a motion sensor which may include an accelerometer, an acoustic sensor and/or a pressure sensor. Pacing signal generator 124 is configured to deliver electrical stimulation signals to ventricle 21 via electrodes 111, 112.

ILP 10 may include a housing, fixation tines, and the electrodes 111, 112. The housing may have a pill-shaped cylindrical form factor in some examples. Fixation tines are configured to connect (e.g., anchor) ILP 10 to heart 20. Fixation tines may be fabricated from a shape memory material, such as Nitinol. In some examples, fixation tines may connect ILP 10 to heart 20 within one of the chambers of heart 20. For example, as illustrated and described herein with respect to Fig. 1, fixation tines may be configured to anchor ILP 10 to heart 20 within right ventricle 21. Although ILP 10 includes a plurality of fixation tines that are configured to anchor ILP 10 to cardiac tissue in the right ventricle, it is contemplated that a pacemaker according to the present disclosure may be fixed to cardiac tissue in other chambers of a patient's heart 20 using other types of fixation mechanisms.

The communication unit 128 may enable ILP 10 to communicate with other electronic devices, such as a programmer or other external patient monitor. In some examples, the housing may house an antenna for wireless communication. Housing may also include the power source 132.

ILP 10 may include two electrodes 111, 112, although more than two electrodes may be included on a pacemaker in other examples. Electrodes 111, 112 may be spaced apart a sufficient distance to be able to detect various electrical signals generated by the heart 20, such as P-waves generated by atria and QRS complex generated by ventricles. The housing houses electronic components of ILP 10. Electronic components may include any discrete and/or integrated electronic circuit components that implement analog and/or digital circuits capable of producing the functions attributed to ILP 10 described above.

Processing unit 120 may control pacing signal generator 124 to generate and deliver electrical stimulation to ventricle 21 via electrodes 111, 112. Electrical stimulation may include pacing pulses. Processing unit 120 may control pacing signal generator 124 to deliver electrical stimulation therapy according to one or more therapy programs including pacing parameters, which may be stored in data memory 122.

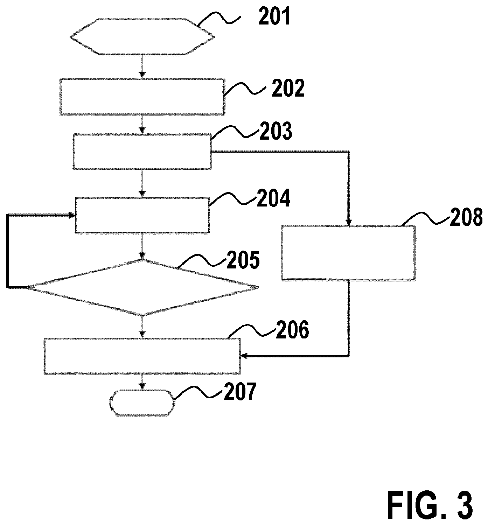

Detector 126 may include circuits that acquire electrical signals (e.g. electric depolarization signals) from the heart including intrinsic cardiac signals, such as intrinsic ventricular signals and/or intrinsic atrial signals. Further, the detector 126 comprises an accelerometer as a motion sensor for determining activity signals of the patient 30 over time. Detector 126 may filter, amplify, and digitize the acquired electrical signals to generate raw digital data. Processing unit 120 may receive the time-dependent digitized data generated by detector 126, in particular the digitized activity signals provided by detector 126. From the activity signals the processing unit 120 determines a pacing rate if the ILP 10 is in the activity-based rate responsive pacing mode which may be set by the programmer. In the activity-based rate responsive pacing mode the processing unit 120 may work in two different modes, namely in the adaption mode and in the stabilized mode. This is explained in the following with reference to the flowchart depicted in Fig. 3.

Processing unit 120 may assess the raw accelerometer signals (activity signals) received from the detector 126 and is configured to set the gain value to a pre-defined initial value which is close to the lowest setting and stored in the data memory 122 after the ILP 10 is implanted (see step 201 in Fig. 3). The processing unit 120 is initially in the adaption mode. After implantation the gain value adaptation algorithm of the adaption mode is running (see step 202 in Fig. 3), wherein the pacing rate is calculated and the gain value (with the components slope and (if applicable) offset) is determined and adapted as indicated above.

In the proposed adaption mode, when the above explained pacing rate and gain value adaptation algorithm is running, the average value of the gain values is calculated from userdefined (or programmable) window size and tracked (see step 203 in Fig. 3), i.e. the average value (e.g. the arithmetic mean value) is determined from the gain values of a pre-defined time period, for example 10 days. Then the delta between the averaged gain values in adjacent windows, e.g. for two adjacent time periods of 10 days, is calculated (see step 204 in Fig. 3) and is compared with a pre-defined stability threshold value (stored in the data memory 122, see step 205 in Fig 3). If the delta is smaller than the stability threshold value, the determined gain value is mostly stabilized and only changes within small variations. In this case, the gain value is locked to the stable value (see step 206 in Fig. 3), e.g. the average gain value determined in the most recent window, which is considered to be the mostly appropriate gain value for the activity level of the patient 30. This locked gain value is stored in the data memory 122, and the rate/gain adaptation algorithm does not need to run in the background anymore. The final step (step 207) is reached. When the pacemaker is programmed to work in rate-adaptive pacing mode, the locked gain value is used in the stabilized mode as the scaling factor and, if applicable, the offset value to convert a motion signal determined as indicated above to the pacing rate. As further depicted in Fig. 3 in addition to using the delta between average gain values to determine the stabilization of the gain values, this algorithm may also be run for a pre-defined adaption time period (userdefined and/or programmable) to ensure the gain value reaches the stable stage (see step 208 in Fig. 3) and then lock the gain value into the stabilized gain value (see step 206 in Fig. 3). The processing unit 120 then transitions to the stabilized mode.

If the delta is equal to or bigger than the stability threshold value (see step 205 in Fig. 3), the delta value of the average of the next adjacent windows is calculated in step 204 of Fig. 3 and so on.

In addition, the stability of the locked gain value may be checked and updated at regular intervals, for example, every 6 months, or per the request of the patient. The update of the locked gain value is similar to the initial adaption and locking of the gain value. The only difference is that in the update procedure, the initial gain value is the previously locked value instead of initial gain value. This means that the algorithm is restarted with step 202 of Fig. 3, wherein the initial gain value is the previously locked gain value. Various triggers may induce unlocking the gain value, including timer, manual clinician settings, expiry of periods of time whereby pacing rates had fallen within a high or low heart rate range, detection of changes in metabolism, behavioral, or medication trends, and other clinically relevant signals.

The processing unit 120 continuously determines a pacing control signal, for example for ventricular pacing, in the activity-based rate responsive pacing algorithm based on the pacing rate calculated using the actual gain value (in the adaption mode) or using the locked gain value (in the stabilized mode) based on the activity signal received from the detector 126 as explained above. The pacing control signal is transmitted to the pacing signal generator 124 which produces the corresponding electrical pacing pulses which will then applied by the electrodes 111, 112 to the patient's heart 20, for example the right ventricle 21.

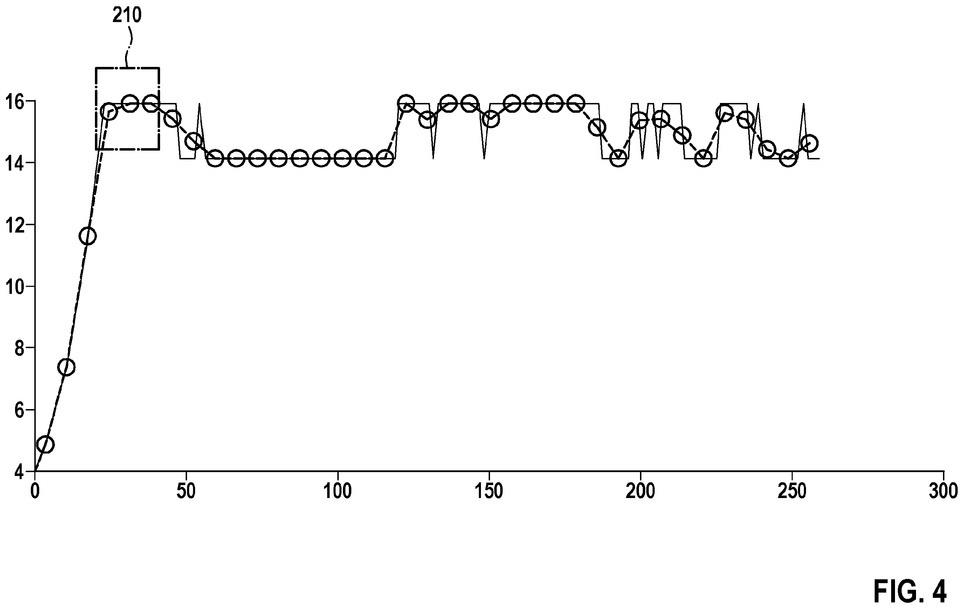

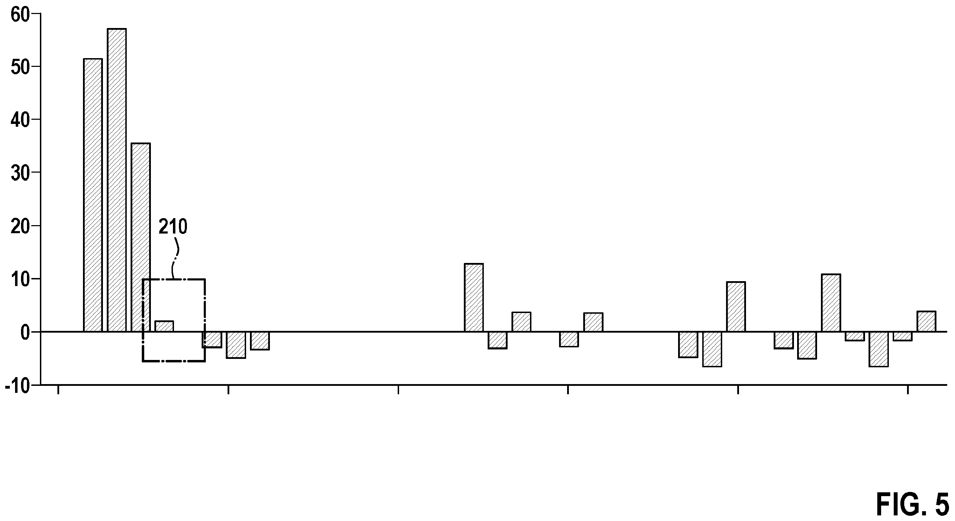

The gain value is locked in the stabilized mode and the rate adaptation can be turned off so that the current budget of ILP 10 is met. Fig. 4 shows an example of the gain value (slope component) over time in a patient after implantation. Further, Fig. 5 shows the delta of the average gain values between two adjacent windows. From Fig. 4 it may be derived, that the gain value is incremented in the adaption mode from the initial pre-defined value for a certain period, then remains stable with a small variation. This represents a typical trend of adapted gain values in the implanted pacemakers. In this example, the gain settings have the step size of approximately 12.5%. From Fig. 5 it may be derived, in the adaption period of the gain value (i.e. in the adaption mode), the average gain value is changed by more than 25% (2 step sizes). On the other hand, when the gain value enters the stable period, the delta between the average gain values in adjacent windows is mostly within 12.5% (1 step size). Therefore, the average gain value is a good parameter to determine the stabilization of the gain value. Then, after the gain value is stabilized, the gain value can be locked and stored in the register of the device (i.e. the processing unit 120 transitions into the stabilized mode), while the rate adaptation algorithm as well as the motion sensor doesn't need to be run in the background to support the gain value update. The transition into the stabilized mode is not shown in Fig. 4 and 5. In Fig. 4 and 5 a time window is represented by a rectangle 210.

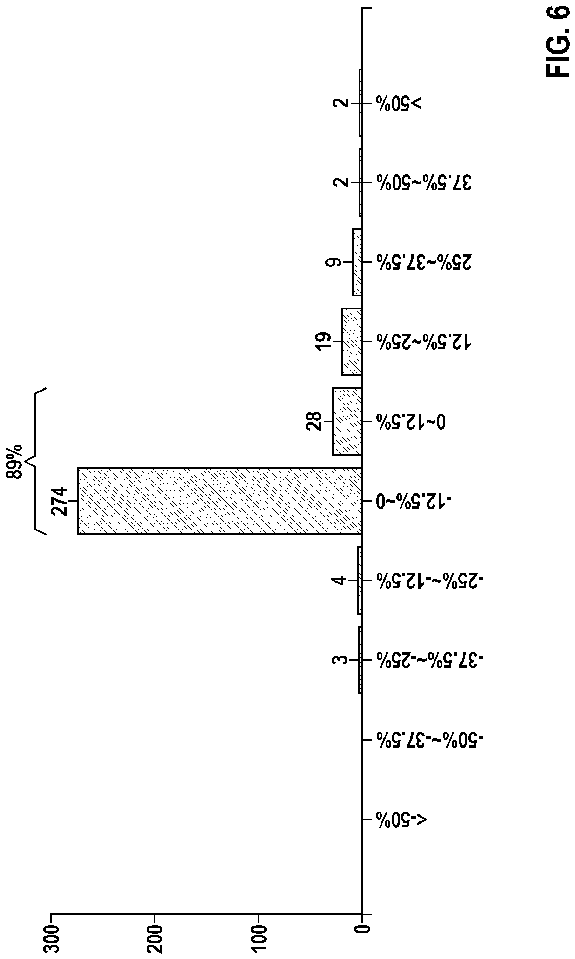

In order to ensure the locked gain value can represent the appropriate gain value corresponding to the activity level of the patient, the variability of the gain value after the transition into the stabilized mode was analyzed using the data from VVIR traditional pacemakers. The example is shown in Fig. 6. The variability was calculated by the difference between the locked value of the gain and the expected value of the gain from the adaption mode. The variability may be used to evaluate the locked gain may represent the actual gain value if the adaption mode keeps running. Thus, the locked gain is compared with the expected value of the gain from the adaption mode. Fig 6 shows the number of cases in which the gain value changes with regard to the locked gain value vs. the change interval. It should be noted that in this example, the step size of the gain value settings is 12.5%. Thus, for most of the patients, after the locking point (i.e. the transition from the adaption mode into the stabilized mode), the obtained gain value from the continuously running rateadaptation is only 1 step different from the locked gain value. Therefore, using the proposed method, the locked gain value can indeed represent the appropriate gain value corresponding to the activity level of the patient.

The above described inventive pacemaker and operation method may further be described by the following general features:
- Tracking average gain in the adaption mode until it meets the stability criterion, namely the delta is small enough and/or until the algorithm is running for a certain period.
- The delta between the average gain values in adjacent windows calculated in the adaption mode is indicative of the stabilization of gain value.
- After the gain value is stabilized, the processing unit 120 transitions into the stabilized mode and the gain value is locked to the average gain value in the last window. This gain value is also referred to as locked gain value.
- In the stabilized mode, motion sensor of the detector 126 (e.g. the accelerator) and the rate adaptation algorithm is turned off when the pacemaker is not in rate adaptive pacing mode to reduce the current consumption and improve the longevity of the ILP or the conventional pacemaker. When the pacemaker is in rate-adaptive pacing mode, the locked gain value is used to calculate the pacing rate.
- The stabilized gain value may be updated per request or at the scheduled follow-up(s).
- Clinical follow-up(s) or timers, other clinically relevant events may also revert the pacer back to adaptation mode, allowing for further adaptation and determination of a new stabilized gain value.
- It is also possible to lock to multidimensional gain values, e.g. one gain value for typical resting days and/or typical exercising days and/or typical sleeping periods and typical active periods. Upon the selection of the patients, different locked gain values may be used to support rate adaptation algorithm.

The above described pacemaker and operation method realize the following advantages:
- The pacemaker has reduced current consumption, meets the current budget of leadless pacer and extends the longevity.
- It is proven that the locked gain value is a robust gain value, not susceptible to random noise in the activity level.
- By programming the time window for averaging the gain values, the locking strategy is flexible to lock to the most appropriate gain value for the patients with different activity patterns.
- The above method is readily to be customized. For instance, multidimensional gain values may be locked as indicated above. Further, gain values with two components (slope and offset) may be locked, as well. Upon the selection of the patients, different locked values may be used to support rate adaptation algorithm.
- The inventive operation method is simple to implement.

## Claims

1. A pacemaker (10) for a patient's heart (20), comprising a processing unit (120), a detector (126) and a pacing signal generator (124), wherein the processing unit (120), the detector (126) and the pacing signal generator (124) are electrically interconnected, wherein the detector (126) is configured to determine activity signals of the patient and to provide the activity signals to the processing unit (126), wherein the processing unit (126) is configured to determine a pacing rate based on the currently received activity signals of the detector (126) and on a gain value in an adaption mode or in a stabilized mode, wherein the processing unit (126) is configured to produce a pace control signal based on the determined pacing rate and to provide it to the pacing signal generator (124), wherein in the adaption mode the processing unit (120) is configured to adapt the gain value to the specific patient, wherein the processing unit (120) is configured to stay in the adaption mode as long as at least one stability criterion is not met and to transition in the stabilized mode if the processing unit (120) identifies that the at least one stability criterion is met, wherein in the stabilized mode the processing unit (120) is configured to use a locked gain value determined based on the most recently adapted gain values for determining the pacing rate.

2. The pacemaker of claim 1, wherein the gain value is a 1-dimensional or multidimensional value.

3. The pacemaker of any of the previous claims, wherein the stability criterion is met if an absolute value of a difference of a first average value of the adapted gain values determined within a first time period and a second average value of the adapted gain values determined within a second time period is less than a pre-determined stability threshold value and/or if the pacing rate is determined in the adaption mode during a pre-defined adaption time period.

4. The pacemaker of the previous claim, wherein the first time period and the second time period are directly adjacent, most recently assessed time periods.

5. The pacemaker of any of the previous claims, wherein in the stabilized mode the processing unit (120) is configured to interrupt or reduce detecting the activity signals by the detector (126), wherein the processing unit (120) is configured to determine a motion signal from the reduced activity signals.

6. The pacemaker of any of the previous claims, wherein the processing unit (120) transitions from the stabilized mode into the adaption mode if the processing unit (120) receives a respective request and/or if a pre-defined third time period is expired since the transition into the stabilized mode.

7. The pacemaker of any of the previous claims, wherein the pacemaker (10) works in the VDD mode.

8. An operation method of a pacemaker (10) for a patient's heart (20), wherein the pacemaker comprises a processing unit (120), a detector (126) and a pacing signal generator (124), wherein the processing unit (120), the detector (126) and the pacing signal generator (124) are electrically interconnected, wherein activity signals of the patient are determined by the detector (126) and transmitted to the processing unit (120), wherein a pacing rate is determined by the processing unit (120) based on the current activity signals of the detector (126) and based on a gain value in an adaption mode or in a stabilized mode, wherein a pace control signal is produced by the processing unit (120) based on the determined pacing rate and transmitted to the pacing signal generator (124), wherein in the adaption mode the gain value is adapted to the specific patient, wherein the processing unit (120) stays in the adaption mode as long as at least one stability criterion is not met and transitions in the stabilized mode if the processing unit (120) identifies that the at least one stability criterion is met, wherein in the stabilized mode the processing unit (120) uses a locked gain value determined based on the most recently adapted gain values for determining the pacing rate.

9. The method of claim 8, wherein the gain value is a 1-dimensional or multidimensional value.

10. The method of any of the claims 8 to 9, wherein the stability criterion is met if an absolute value of a difference of a first average value of the adapted gain values determined within a first time period and a second average value of the adapted gain values determined within a second time period is less than a pre-determined stability threshold value and/or if the pacing rate is determined in the adaption mode during a pre-defined adaption time period.

11. The method of claim 10, wherein the first time period and the second time period are directly adjacent, most recently assessed time periods.

12. The method of any of the claims 8 to 11, wherein in the stabilized mode detection of the activity signals by the detector (126) is interrupted or reduced by the processing unit (120), wherein the processing unit (120) determines a motion signal from the reduced activity signal.

13. The method of any of the claims 8 to 12, wherein a transition from the stabilized mode into the adaption mode is provided by the processing unit (120) if the processing unit (120) receives a respective request and/or if a pre-defined third time period is expired since the transition into the stabilized mode.

14. A computer program product comprising instructions which, when executed by a processing unit (120), cause the processing unit (120) to perform the steps of the method according to any of the claims 8 to 13.

15. Computer readable data carrier storing a computer program product according to claim 14.

## Patentansprüche

1. Herzschrittmacher (10) für ein Herz (20) eines Patienten, umfassend eine Verarbeitungseinheit (120), einen Detektor (126) und einen Stimulationssignal-Generator (124), wobei die Verarbeitungseinheit (120), der Detektor (126) und der Stimulationssignal-Generator (124) elektrisch miteinander verbunden sind, wobei der Detektor (126) dazu ausgebildet ist, Aktivitätssignale des Patienten zu bestimmen und die Aktivitätssignale der Verarbeitungseinheit (126) bereitzustellen, wobei die Verarbeitungseinheit (126) dazu ausgebildet ist, eine Stimulationsrate auf Grundlage der aktuell empfangenen Aktivitätssignale des Detektors (126) und eines Gain-Werts in einem Adaptionsmodus oder in einem Stabilisierungsmodus zu bestimmen, wobei die Verarbeitungseinheit (126) dazu ausgebildet ist, auf Grundlage der bestimmten Stimulationsrate ein Stimulationskontrollsignal zu erzeugen und dieses dem Stimulationssignal-Generator (124) bereitzustellen, wobei im Adaptionsmodus die Verarbeitungseinheit (120) dazu ausgebildet ist, den Gain-Wert an den spezifischen Patienten anzupassen, wobei die Verarbeitungseinheit (120) dazu ausgebildet ist, im Adaptionsmodus zu verbleiben, solange mindestens ein Stabilitätskriterium nicht erfüllt ist, und in den Stabilisierungsmodus zu wechseln, wenn die Verarbeitungseinheit (120) erkennt, dass das mindestens eine Stabilitätskriterium erfüllt ist, wobei im Stabilisierungsmodus die Verarbeitungseinheit (120) dazu ausgebildet ist, zur Bestimmung der Stimulationsrate einen gesperrten Gain-Wert zu verwenden, der auf Grundlage der zuletzt angepassten Gain-Werte bestimmt wurde.

2. Herzschrittmacher nach Anspruch 1, wobei der Gain-Wert ein eindimensionaler oder mehrdimensionaler Wert ist.

3. Herzschrittmacher nach einem der vorhergehenden Ansprüche, wobei das Stabilitätskriterium erfüllt ist, wenn ein Absolutwert einer Differenz zwischen einem ersten Mittelwert der angepassten Gain-Werte, der innerhalb eines ersten Zeitraums bestimmt wurde, und einem zweiten Mittelwert der angepassten Gain-Werte, der innerhalb eines zweiten Zeitraums bestimmt wurde, kleiner ist als ein vorbestimmter Stabilitätsschwellenwert und/oder wenn die Stimulationsrate im Adaptionsmodus während eines vorbestimmten Adaptionszeitraums bestimmt wird.

4. Herzschrittmacher nach dem vorhergehenden Anspruch, wobei der erste Zeitraum und der zweite Zeitraum unmittelbar aufeinanderfolgende, zuletzt ausgewertete Zeiträume sind.

5. Herzschrittmacher nach einem der vorhergehenden Ansprüche, wobei im Stabilisierungsmodus die Verarbeitungseinheit (120) dazu ausgebildet ist, das Erfassen der Aktivitätssignale durch den Detektor (126) zu unterbrechen oder zu reduzieren, wobei die Verarbeitungseinheit (120) dazu ausgebildet ist, aus den reduzierten Aktivitätssignalen ein Bewegungssignal zu bestimmen.

6. Herzschrittmacher nach einem der vorhergehenden Ansprüche, wobei die Verarbeitungseinheit (120) dazu ausgebildet ist, vom Stabilisierungsmodus in den Adaptionsmodus überzugehen, wenn die Verarbeitungseinheit (120) eine entsprechende Anforderung empfängt und/oder wenn seit dem Übergang in den Stabilisierungsmodus ein vorbestimmter dritter Zeitraum abgelaufen ist.

7. Herzschrittmacher nach einem der vorhergehenden Ansprüche, wobei der Herzschrittmacher (10) dazu ausgebildet ist im VDD-Modus zu arbeiten.

8. Betriebsverfahren eines Herzschrittmachers (10) für ein Herz (20) eines Patienten, wobei der Herzschrittmacher eine Verarbeitungseinheit (120), einen Detektor (126) und einen Stimulationssignal-Generator (124) umfasst, wobei die Verarbeitungseinheit (120), der Detektor (126) und der Stimulationssignal-Generator (124) elektrisch miteinander verbunden sind, wobei Aktivitätssignale des Patienten durch den Detektor (126) bestimmt und an die Verarbeitungseinheit (120) übertragen werden, wobei eine Stimulationsrate durch die Verarbeitungseinheit (120) auf Grundlage der aktuellen Aktivitätssignale des Detektors (126) und auf Grundlage eines Gain-Werts in einem Adaptionsmodus oder in einem Stabilisierungsmodus bestimmt wird, wobei ein Stimulationskontrollsignal durch die Verarbeitungseinheit (120) auf Grundlage der bestimmten Stimulationsrate erzeugt und an den Stimulationssignal-Generator (124) übertragen wird, wobei im Adaptionsmodus der Gain-Wert an den spezifischen Patienten angepasst wird, wobei die Verarbeitungseinheit (120) im Adaptionsmodus verbleibt, solange mindestens ein Stabilitätskriterium nicht erfüllt ist, und in den Stabilisierungsmodus übergeht, wenn die Verarbeitungseinheit (120) erkennt, dass das mindestens eine Stabilitätskriterium erfüllt ist, wobei im Stabilisierungsmodus die Verarbeitungseinheit (120) zur Bestimmung der Stimulationsrate einen gesperrten Gain-Wert verwendet, der auf Grundlage der zuletzt angepassten Gain-Werte bestimmt wurde.

9. Verfahren nach Anspruch 8, wobei der Gain-Wert ein eindimensionaler oder mehrdimensionaler Wert ist.

10. Verfahren nach einem der Ansprüche 8 bis 9, wobei das Stabilitätskriterium erfüllt ist, wenn ein Absolutwert einer Differenz zwischen einem ersten Mittelwert der angepassten Gain-Werte, der innerhalb eines ersten Zeitraums bestimmt wurde, und einem zweiten Mittelwert der angepassten Gain-Werte, der innerhalb eines zweiten Zeitraums bestimmt wurde, kleiner ist als ein vorbestimmter Stabilitätsschwellenwert und/oder wenn die Stimulationsrate im Adaptionsmodus während eines vorbestimmten Adaptionszeitraums bestimmt wird.

11. Verfahren nach Anspruch 10, wobei der erste Zeitraum und der zweite Zeitraum unmittelbar aufeinanderfolgende, zuletzt ausgewertete Zeiträume sind.

12. Verfahren nach einem der Ansprüche 8 bis 11, wobei im Stabilisierungsmodus das Erfassen der Aktivitätssignale durch den Detektor (126) durch die Verarbeitungseinheit (120) unterbrochen oder reduziert wird, wobei die Verarbeitungseinheit (120) aus dem reduzierten Aktivitätssignal ein Bewegungssignal bestimmt.

13. Verfahren nach einem der Ansprüche 8 bis 12, wobei ein Übergang vom Stabilisierungsmodus in den Adaptionsmodus durch die Verarbeitungseinheit (120) bereitgestellt wird, wenn die Verarbeitungseinheit (120) eine entsprechende Anforderung empfängt und/oder wenn seit dem Übergang in den Stabilisierungsmodus ein vorbestimmter dritter Zeitraum abgelaufen ist.

14. Computerprogrammprodukt, das Anweisungen umfasst, die, wenn sie durch eine Verarbeitungseinheit (120) ausgeführt werden, die Verarbeitungseinheit (120) veranlassen, die Schritte des Verfahrens nach einem der Ansprüche 8 bis 13 auszuführen.

15. Computerlesbarer Datenträger, der ein Computerprogrammprodukt nach Anspruch 14 speichert.

## Revendications

1. Stimulateur cardiaque (10) destiné au cœur d'un patient (20), comprenant une unité de traitement (120), un détecteur (126) et un générateur de signal de stimulation (124), dans lequel l'unité de traitement (120), le détecteur (126) et le générateur de signal de stimulation (124) sont interconnectés électriquement, dans lequel le détecteur (126) est configuré pour déterminer des signaux d'activité du patient et pour fournir les signaux d'activité à l'unité de traitement (126), dans lequel l'unité de traitement (126) est configurée pour déterminer une fréquence de stimulation en se basant sur les signaux d'activité actuellement reçus du détecteur (126) et sur une valeur de gain dans un mode d'adaptation ou dans un mode stabilisé, dans lequel l'unité de traitement (126) est configurée pour produire un signal de commande de stimulation en se basant sur la fréquence de stimulation déterminée et pour le fournir au générateur de signaux de stimulation (124), dans lequel dans le mode d'adaptation l'unité de traitement (120) est configurée pour adapter la valeur de gain au patient spécifique, dans lequel l'unité de traitement (120) est configurée pour rester dans le mode d'adaptation aussi longtemps qu'au moins un critère de stabilité n'est pas atteint et pour passer au mode stabilisé si l'unité de traitement (120) identifie que l'au moins un critère de stabilité est atteint, dans lequel dans le mode stabilisé l'unité de traitement (120) est configurée pour utiliser une valeur de gain verrouillée déterminée en se basant sur les valeurs de gain les plus récemment adaptées pour déterminer la fréquence de stimulation.

2. Stimulateur cardiaque selon la revendication 1, dans lequel la valeur de gain est une valeur monodimensionnelle ou multidimensionnelle.

3. Stimulateur cardiaque selon l'une quelconque des revendications précédentes, dans lequel le critère de stabilité est atteint si une valeur absolue d'une différence d'une première valeur moyenne des valeurs de gain adaptées déterminées au sein d'une première période et une seconde valeur moyenne des valeurs de gain adaptées déterminées au sein d'une deuxième période est inférieure à une valeur de seuil de stabilité prédéterminée et/ou si la fréquence de stimulation est déterminée dans le mode d'adaptation pendant une période d'adaptation prédéfinie.

4. Stimulateur cardiaque selon la revendication précédente, dans lequel la première période et la deuxième période sont des périodes le plus récemment évaluées et directement adjacentes.

5. Stimulateur cardiaque selon la revendication précédente, dans lequel dans le mode stabilisé l'unité de traitement (120) est configurée pour interrompre ou réduire la détection des signaux d'activité par le détecteur (126), dans lequel l'unité de traitement (120) est configurée pour déterminer un signal de mouvement à partir des signaux d'activité réduite.

6. Stimulateur cardiaque selon l'une quelconque des revendications précédentes, dans lequel l'unité de traitement (120) est configurée pour passer du mode stabilisé au mode d'adaptation si l'unité de traitement (120) reçoit une requête respective et/ou si une troisième période prédéfinie est terminée depuis le passage au mode stabilisé.

7. Stimulateur cardiaque selon l'une quelconque des revendications précédentes, dans lequel le stimulateur cardiaque (10) est configurée pour fonctionner en mode VDD.

8. Procédé de fonctionnement d'un stimulateur cardiaque (10) destiné au cœur d'un patient (20), dans lequel le stimulateur cardiaque comprend une unité de traitement (120), un détecteur (126) et un générateur de signal de stimulation (124), dans lequel l'unité de traitement (120), le détecteur (126) et le générateur de signal de stimulation (124) sont interconnectés électriquement, dans lequel les signaux d'activité du patient sont déterminés par le détecteur (126) et transmis à l'unité de traitement (120), dans lequel une fréquence de stimulation est déterminée par l'unité de traitement (120) en se basant sur les signaux d'activité actuels du détecteur (126) et en se basant sur une valeur de gain dans un mode d'adaptation ou dans un mode stabilisée, dans lequel un signal de commande de stimulation est produit par l'unité de traitement (120) en se basant sur la fréquence de stimulation déterminée et transmis au générateur de signaux de stimulation (124), dans lequel dans le mode d'adaptation la valeur de gain est adaptée au patient spécifique, dans lequel l'unité de traitement (120) reste dans le mode d'adaptation aussi longtemps qu'au moins un critère de stabilité n'est pas atteint et passe en mode stabilisé si l'unité de traitement (120) identifie que l'au moins un critère de stabilité est atteint, dans lequel dans le mode stabilisé l'unité de traitement (120) utilise une valeur de gain verrouillée déterminée en se basant sur les valeurs de gain le plus récemment adaptées pour déterminer la fréquence de stimulation.

9. Procédé selon la revendication 8, dans lequel la valeur de gain est une valeur monodimensionnelle ou multidimensionnelle.

10. Procédé selon l'une quelconque des revendications 8 à 9, dans lequel le critère de stabilité est atteint si une valeur absolue d'une différence d'une première valeur moyenne des valeurs de gain adaptées déterminées au sein d'une première période et une seconde valeur moyenne des valeurs de gain adaptées déterminées au sein d'une deuxième période est inférieure à une valeur de seuil de stabilité prédéterminée et/ou si la fréquence de stimulation est déterminée dans le mode d'adaptation pendant une période d'adaptation prédéfinie.

11. Procédé selon la revendication 10, dans lequel la première période et la deuxième période sont des périodes le plus récemment évaluées et directement adjacentes.

12. Procédé selon l'une quelconque des revendications 8 à 11, dans lequel dans le mode stabilisé la détection des signaux d'activité par le détecteur (126) est interrompue ou réduite par l'unité de traitement (120), dans lequel l'unité de traitement (120) détermine un signal de mouvement à partir du signal d'activité réduite.

13. Procédé selon l'une quelconque des revendications 8 à 12, dans lequel un passage du mode stabilisé au mode d'adaptation est fourni par l'unité de traitement (120) si l'unité de traitement (120) reçoit une requête respective et/ou si une troisième période prédéfinie est terminée depuis le passage au mode stabilisé.

14. Produit de programme informatique comprenant des instructions qui, lorsqu'elles sont exécutées par une unité de traitement (120), amènent l'unité de traitement (120) à réaliser les étapes du procédé selon l'une quelconque des revendications 8 à 13.

15. Support de données lisible par ordinateur stockant un produit de programme informatique selon la revendication 14.
